# EUROPÄISCHE PATENTANMELDUNG

(11) **EP 1 475 433 A1**
(43) Veröffentlichungstag der Anmeldung: **10.11.2004**
(21) Anmeldenummer: 03010206.5
(22) Anmeldetag: 06.05.2003
(51) Int. Cl.: C12N 5/00

(54) **Transfektionsverfahren für Säugerzellen**

(71) Anmelder: GBF Gesellschaft für Biotechnologische Forschung mbH, 38124 Braunschweig (DE)
(72) Erfinder: Wagner, Roland, 38159 Vechelde (DE); Beer, Christiane, 38118 Braunschweig (DE); Bassani Molinas, Maria de los Milagros, 38124 Braunschweig (DE); Wirth, Manfred, 38300 Wolfenbüttel (DE)
(74) Vertreter: VOSSIUS & PARTNER

(57) **Zusammenfassung**

Die Erfindung betrifft ein Verfahren zur transienten Expression von (Poly)peptiden in Säugerzellen, wobei man die folgenden Schritte durchführt: (a) Züchtung von Säugerzellen in einem Medium, das aus Calcium-freiem, mit Vitaminen und Aminosäuren supplementiertem DMEM und Freestyle™ Medium in einem Volumenverhältnis von 30:70 bis 70:30 besteht; (b) Transfektion der in Schritt (a) genannten Säugerzellen mit einem das (Poly)peptid kodierenden und exprimierbaren Nucleinsäuremolekül in Calcium-freiem DMEM unter Verwendung von Polyethylenimin; und (c) Züchtung der in Schritt (b) transfizierten Säugerzellen. Bevorzugt ist dabei, dass die Transfektion in Schritt (b) in einem Pilot- oder Produktionsbioreaktor durchgeführt wird. Ferner betrifft die Erfindung ein Kulturmedium, das das aus Calcium-freiem, mit Vitaminen und Aminosäuren supplementiertem DMEM und Freestyle™ Medium in einem Volumenverhältnis von 30:70 bis 70:30 besteht.

## Beschreibung

Die Erfindung betrifft ein Verfahren zur transienten Expression von (Poly)peptiden in Säugerzellen, wobei man die folgenden Schritte durchführt: (a) Züchtung von Säugerzellen in einem Medium, das aus Calcium-freiem, mit Vitaminen und Aminosäuren supplementiertem DMEM und Freestyle™ Medium in einem Volumenverhältnis von 30:70 bis 70:30 besteht; (b) Transfektion der in Schritt (a) genannten Säugerzellen mit einem das (Poly)peptid kodierenden und exprimierbaren Nucleinsäuremolekül in Calcium-freiem DMEM unter Verwendung von Polyethylenimin; und (c) Züchtung der in Schritt (b) transfizierten Säugerzellen. Bevorzugt ist dabei, dass die Transfektion in Schritt (b) in einem Pilot- oder Produktionsbioreaktor durchgeführt wird. Ferner betrifft die Erfindung ein Kulturmedium, das das aus Calcium-freiem, mit Vitaminen und Aminosäuren supplementiertem DMEM und Freestyle™ Medium in einem Volumenverhältnis von 30:70 bis 70:30 besteht.

In der Anmeldung ist eine Anzahl von Dokumenten aus dem Stand der Technik genannt. Der Offenbarungsgehalt dieser Dokumente, einschließlich Betriebsanweisungen von Herstellern wird hiermit per Bezugnahme in die vorliegende Anmeldung inkorporiert.

Verglichen mit mikrobiellen Expressionssystemen ist die Proteinexpression in Säugerzellen langwierig, wobei der zeitaufwendigste Teil die Herstellung der stabil produzierenden Zelllinie ist. Dagegen erlaubt die transiente Genexpression die Proteinsynthese sofort nach dem Gentransfer ohne die stabile Integration in die chromosomale DNA. Bisher blieb bei der transienten Transfektion immer der Nachteil, dass entweder eine geringe Menge Serum zum effizienten Gentransfer nötig war oder ein Waschschritt eingeführt werden musste, der unerwünschte, die Transfektionseffizienz behindernde Faktoren, eliminierte.

Die Gegenwart von Komponenten tierischer Herkunft, insbesondere von Serum, ist jedoch für die Herstellung von Therapeutika, die mit Hilfe rekombinanter Säugerzelllinien produziert werden, unerwünscht. Die Notwendigkeit eines Wachsschrittes dagegen behindert die Scale-up-Fähigkeit des Prozesses in den Pilot- und Produktionsmaßstab, da ein Zellwaschprozess die vollständige Ernte der Kulturbrühe bedingt und damit, neben dem erheblichen Zeitaufwand, ein unkalkulierbares Kontaminationsrisiko darstellt.

So beschreiben Durocher et al., Nucl. Acids Res. 30 (2002), e9 ein System zur transienten Expression menschlichen embryonischen Nierenzellen der Zellinie 293 mittels Polyethylenimin (PEI) als Transfektionsagens. Als einer der wesentlichen Parameter für eine Züchtung der transfizierten Zellen in großem Maßstab wird das Züchtungsmedium diskutiert. Bekannt war unter anderem, dass das Vorhandensein von Serum im Medium die Transfektionseffizienz negativ beeinflussen kann. Diesem Nachteil wurde dadurch begegnet, dass nach der Anzucht der Zellen und vor der Transfektion serumhaltiges durch serumfreies Medium ausgetauscht wurde. Durocher und Kollegen fanden nun, dass die Transfektionseffizienz nicht behindert wurde und sogar gesteigert werden konnte, wenn die Transfektion in konditioniertem und damit proteinhaltigem Medium durchgeführt wurde. Sie schlossen aus diesen Ergebnissen, dass eine einen Transfektionsschritt mit PEI einschließende Kultivierung derartiger Zelllinien in großem Maßstab vorteilhafterweise ohne einen Zwischenschritt durchgeführt werden kann, der während der Transfektion die Züchtung in serumfreiem Medium und damit einen Medienwechsel erfordert. Der Nachteil dieses Verfahrens liegt darin begründet, dass die Zellen in serumhaltigem Medium gezüchtet werden, was einen erhöhten Aufwand bei der Weiterverarbeitung dieser Zellen für pharmazeutische Zwecke bzw. wie bereits erwähnt Probleme bei der Zulassung derartiger Produkte als Arzneimittel mit sich bringen kann.

Im Gegensatz zu Durocher und Kollegen fanden Schlaeger und Christensen (Cytotechnology 30 (1999), 71-83), dass konditioniertes Medium einen negativen Einfluss auf die Transfektionseffizienz hat. Sie wählten für ein Kultivierungsverfahren von mittels PEI als Transfektionsagens transfizierten Zellen serumfreies Medium, dem Heparin zugesetzt wurde, um das Verklumpen der Zellen zu Zellaggregaten zu minimieren. Da Heparin die DNA-Aufnahme in die Zellen jedoch vollständig verhindert, muss bei der Züchtung der Zellen in großem Maßstab vor der Transfektion ein Waschschritt zur Entfernung von Heparin durchgeführt werden. Darüber hinaus musste bei der Züchtung in serumfreiem Medium diesem ein Proteinhydrolysat wie Primatone zugesetzt werden. Somit bringt das von Schlaeger und Christensen beschriebene Verfahren die Nachteile mit sich, dass Waschschritte durchzuführen sind, die ein "Upscaling" des Züchtungsverfahren wesentlich erschweren. Darüber hinaus kann auch das Proteinlysat im Medium Probleme bei der Aufreinigung und Zulassung mit sich bringen. Aufgabe der vorliegenden Erfindung war daher, ein Verfahren bereitzustellen, das diese Nachteile aus dem Stand der Technik vermeidet und ein effizientes, kostengünstiges und mit wenig Zeitaufwand durchzuführendes Verfahren zur Transfektion und nachfolgenden Züchtung von Zellen im industriellen Maßstab darstellt. Diese Aufgabe wird durch die in den Ansprüchen bereitgestellten Ausführungsformen gelöst.

Somit betrifft die Erfindung ein Verfahren zur transienten Expression von (Poly)peptiden in Säugerzellen, wobei man die folgenden Schritte durchführt:
(a) Züchtung von Säugerzellen in einem Medium, das vollständig oder im wesentlichen aus Calcium-freiem, mit Vitaminen und Aminosäuren supplementiertem DMEM und Freestyle™ Medium in einem Volumenverhältnis von 30:70 bis 70:30 besteht;
(b) Transfektion der in Schritt (a) genannten Säugerzellen mit einem das (Poly)peptid kodierenden und exprimierbaren Nucleinsäuremolekül in Calcium-freiem DMEM unter Verwendung von Polyethylenimin; und
(c) Züchtung der in Schritt (b) transfizierten Säugerzellen.

Der Begriff "transiente Expression" ist im Stand der Technik bekannt und bezeichnet eine zeitlich begrenze Expression eines Gens. Im Sinne der Erfindung ist der Begriff darüber hinaus dadurch gekennzeichnet, dass die Expression von einem extrachromosomalen Element, wie einem Plasmid oder einem anderen extrachromosomal vorkommenden Element aus gesteuert wird.

Der Begriff "(Poly)peptide" umfasst sowohl Peptide als auch Polypeptide und damit Proteine. Gemäß üblicher Begriffbestimmungen wird als Peptid eine Aminosäurenkette mit einer Länge bis zu 30 Aminosäuren bezeichnent, während Polypeptide eine Aminosäurenkette mit einer Länge von mehr als 30 Aminosäuren aufweisen.

Der Begriff "das vollständig oder im wesentlichen aus [...] besteht" bedeutet im Sinne dieser Erfindung, dass bei Vollständigkeit dem Medium keine weiteren Komponenten zugesetzt sind. Im Falle, dass das Kulturmedium im wesentlichen aus diesen Medien besteht, können dem Kulturmedium in kleineren Mengen noch Bestandteile wie Antibiotika oder produktivitätssteigernde Reagenzien, z.B. Butyrat zugesetzt sein. Auch der Begriff im wesentlichen schließt jedoch aus, dass dem Medium weitere Bestandteile anderer Kulturmedien wie RPMI in signifikanten Mengen, d.h. in Mengen größer als 5%, bevorzugt in Mengen größer als 2% und stärker bevorzugt in Mengen größer als 1% zugesetzt sind. Idealerweise enthält das Kulturmedium keine Bestandteile außer denen der erfindungsgemäß genannten Medien.

Der Begriff "Calcium-frei" bedeutet erfindungsgemäß, dass der Anteil an Calcium im Medium unter 1 µmol/L.

Der Begriff "DMEM" ist eine Abkürzung für "Dulbecco's Modified Eagle Medium" wie im Stand der Technik bekannt. Dieses Medium ist proteinfrei und zusammengesetzt aus einer Energiequelle, vornehmlich Glukose und Glutamin, Mineralsalzen, Bicarbonat-Puffersystem, Aminosäuren, Vitaminen und Polyaminen. Eine genaue Zusammensetzung von DMEM ist in Harlow und Lane, "Antibodies, A Laboratory Manual", CSH Press, Cold Spring Harbor 1988, Tabelle 7.1 auf S. 248-249 angegeben, die hiermit spezifisch per Referenz inkorporiert ist. Es kann käuflich beispielsweise von Invitrogen (Gibco BRL) oder Sigma erworben werden. DMEM wird für das erfindungsgemäße Verfahren, je nach eingesetzter Zelllinie mit einem Vitamincocktail und essentiellen Aminosäuren wie Aspartat oder Glutemat versetzt. Für die Züchtung mit DMEM einsetzbare Vitamin-Lösungen sind z.B. beziehbar von Invitrogen (Gibco BRL) oder Sigma.

"Freestyle™ Medium" ist ein proteinfreies Medium, das von der Firma Invitrogen (Gibco BRL) bezogen werden kann.

Der Begriff "Transfektion" ist im Stand der Technik ebenfalls bekannt und wird hier entsprechend verwendet. Er bezeichnet die Einführung von DNA in nichtbakterielle Wirtszellen und vorzugsweise Säugerzellen durch Inkubation der Zellen mit der DNA. Üblicherweise ist für die Transfektion ein Agens nötig, dass die Transfektion ermöglicht. Solche Agentien schließen Polyethylen imid und Calciumchlorid mit ein. Gegebenenfalls müssen die Zellen für die Transfektion besonders präpariert werden. Entsprechende Protokolle sind im Stand der Technik bekannt und beispielsweise in Sambrook et al., "Molecular cloning, a laboratory manual", 2. Auflage, CSH Press 1989, Cold Spring Harbor, oder "Current Protocols in Molecular Biology" (2001), John Wiley & Sons, New York, beschrieben.

"Exprimierbar" ist das Nukleinsäuremolekül, wenn es unter der Kontrolle geeigneter regulatorischer Sequenzen, die in der zu transfizierenden Zelle funktionell sind, stehen. Zu diesen regulatorischen Sequenzen gehören in jedem Falle Promotoren und gegebenenfalls Enhancer etc. Die Auswahl geeigneter regulatorischer Sequenzen gehört zum Standardrepertoire des Fachmanns.

Das erfindungsgemäß zu exprimierende Nukleinsäuremolekül ist vorzugsweise DNA und kann beispielsweise eine cDNA oder eine genomische DNA sein. Diese kann unter der Kontrolle des eigenen oder eines fremden Promotors stehen.

Wie erwähnt, liegt das erfindungsgemäß einsetzbare Volumenverhältnis der beiden Medien im Bereich von 30:70 bis 70:30. Mit anderen Worten kann das DMEM in einem Anteil von 30 bis 70 % mit Freestyle™ Medium vermischt werden. In einer bevorzugten Ausführungsform werden die beiden Medien zu etwa gleichen Teilen (1:1) vermischt. Das Medium unterstützt das Wachstum von Säugerzellen bis zu einer Konzentration von 7 x 10⁶ pro ml in Batch-Kultur.

Die Züchtung der Säugerzellen findet unter üblichen sterilen Bedingungen in Schritt (a) vorzugsweise im Maßstab von Miktotiterplatten (z.B. mit 24 Vertiefungen) oder kleinen Kulturflaschen (z.B. mit einem Volumen von 20 ml statt und in Schritt (b) in größerem Maßstab, z.B. in Rollerflaschen oder Fermentern bzw. Bioreaktoren statt. Die Säugerzellen können dabei adhärent oder in Suspension wachsen. Bevorzugt ist ferner, dass die Kultur als Batch-Kultur gezüchtet wird, also als diskontinuierliche Kultur, bei der das Nährmedium zum Zeitpunkt t=0 beimpft wird und die Fermentation nach Verbrauch des limitierenden Substrats oder zu einem anderen geeigneten Zeitpunkt beendet wird.

Erfindungsgemäß wurde auf Basis des Polykationen-vermittelten Gentransfers (vgl. Boussif et al., Proc. Natl. Acad. Sci. USA 92 (1995), 7297-7301), d.h. unter Verwendung von Polyethylenimin ein Transfektionssystem entwickelt, dass unter vollständig definierten serumfreien Kulturbedingungen und ohne die Durchführung eines Waschschrittes hohe Expressionsraten erreicht. Das Verfahren basiert auf der Verwendung eines speziellen erfindungsgemäßen Nährkulturmediums, das sich aus einer wie oben angegebenen und in einer bevorzugten Ausführungsform 1:1 Mischung von Calcium-freien DMEM und Freestyle™ Medium zusammensetzt. Diese beiden Medienkomponenten sind kommerziell erhältlich und enthalten keine Proteinbestandteile. Die DNA wird mit Polyethylenimin (PEI), vorzugsweise im Verhältnis 0,5-1 : 1-1,5, besonders bevorzugt 0,75-1 :1, ebenfalls serumfrei in Gegenwart von ausschließlich DMEM-Medium zur Transfektion (0,5 - 1 µg DNA, vorzugsweise 0,75 - 1 µg DNA für eine Zellkonzentration von 5 - 20 X 10⁵ mL⁻¹, vorzugsweise 5-10 X 10⁵ mL⁻¹) vorbereitet.

Durch die Verwendung spezieller, die Transfektion nicht behindernder Nährmedien, kann auf die Durchführung eines Waschschrittes vor der Transfektion, insbesondere im grossen Maßstab, beispielsweise im Bioreaktor, verzichtet werden. Zusammen mit der Verwendung chemisch definierter proteinfreier Nährmedien vereint das erfindungsgemäße Verfahren folgende kompetitive Eigenschaften:
- vollständiger Verzicht auf Komponenten tierischer Herkunft
- Zellen wachsen in Suspension
- hohe Akzeptanz bei Zulassungsbehörden
- einfache Scale-up-Fähigkeit bis zum Produktionsmaßstab

Das erfindungsgemäße Verfahren ist gegenüber den im Stand der Technik bekannten Verfahren, wie vorstehend diskutiert, überlegen, da die bisher beschriebenen Techniken entweder für die Verwendung in Pilot- und Produktionsbioreaktoren ungeeignet sind, weil ein Zellwaschritt vor der eigentlichen Transfektion durchgeführt werden muss oder da sie noch geringe Mengen von Serum verwenden und damit noch Bestandteile tierischer Herkunft enthalten.

Bevorzugt ist erfindungsgemäß ein Verfahren, wobei die Säugerzellen Teil einer Zelllinie sind. Zelllinien sind, wie im Stand der Technik bekannt, permanent in vitro wachsende Zellen mit definiertem Ursprung und charakteristischen Eigenschaften. Im erfindungsgemäßen Verfahren einsetzbare Zelllinien sind beispielsweise entweder von Forschungseinrichtungen frei verfügbar oder von anerkannten Hinterlegungsstellen wie der DMSZ oder der ATCC erhältlich.

Die Zelllinie kann beispielsweise eine von menschlichen Zellen abgeleitete Zelllinie sein. Derartige Zelllinien werden bevorzugt eingesetzt, wenn die am (Poly)peptid durchgeführten post-translationalen Modifikationen denen im Menschen möglichst nahe kommen sollen. Erfindungsgemäß besonders bevorzugt ist jedoch ein Verfahren, wobei die Zelllinie eine von tierischen Zellen abgeleitete Zelllinie ist. Tierische Zellen sind im Gegensatz zu Bakterien in der Lage, das rekombinante Protein nativ zu sekretieren. Säugerzelllinien sind darüber hinaus in der Lage, auch humanidentische posttransrationale Modifikationen, wie Glycosylierungen, an dem naszierenden Protein vorzunehmen.

In einer weiteren bevorzugten Ausführungsform betrifft die Erfindung ein Verfahren, wobei die (Poly)peptide menschliche (Poly)peptide sind. Von besonderem Interesse sind dabei Proteine, die in pharmazeutischem Kontext eingesetzt werden können, wie Interleukine, Interferone oder andere Wachstumsoder Differenzierungsfaktioren, Blutproteine wie Proteine, die in die Blutgerinnung involviert sind wie Gewebsplasminogen-Aktivator oder Antikörper.

Ferner ist ein Verfahren bevorzugt, wobei die Transfektion in Schritt (b) in einem Pilot- oder Produktionsbioreaktor durchgeführt wird, wobei die jeweilige maximale Grösse des Bioreaktors durch die vorhandene Menge an DNA bestimmt wird, was vom Fachmann ohne weiteres ermittelt werden kann.

Erfindungsgemäß können verzweigte wie auch unverzweigte Polyethylenimine eingesetzt werden. Da unverzweigte Polyethylenimine Vorteile in der Transfektionseffizienz aufweisen, sind jedoch Verfahren bevorzugt, in denen das eingesetzte Polyethylenimin ein unverzweigtes Polyethylenimin ist. Besonders bevorzugt ist ferner, dass das Polyethylenimin ein Molekulargewicht von 25 kDa aufweist. Andere im erfindungegemäßen Verfahren einsetzbare Polyethylenimine schließen solche mit einem Molekulargewicht von 800, 60 und 22 kDa ein.

Bevorzugt ist erfindungsgemäß auch ein Verfahren, bei dem die Aminosäuren, mit denen das DMEM supplementiert ist, Aspartat und Glutamat sind. Die Aminosäuren werden dem DMEM vorzugsweise in einer Endkonzentration von 80 mg/l Aspartat und 15 mg/l Glutamat zugesetzt.
Die Vitamine werden vorzugsweise in einer Endkonzentration von 4% oder von 1/100 einer vorgefertigten Mischung zugesetzt, wobei die vorgefertigte MEM Vitaminlösung M6895 (Sigma Produktnummer) besonders bevorzugt ist.

Im erfindungsgemäßen Verfahren ist darüber hinaus bevorzugt, dass das (Poly)peptid kodierende Nucleinsäuremolekül vor der Transfektion in einem Verhältnis von mindestens 0,5 µg DNA zu 1,5-2 µg/ml Polyethylenimin mit Calcium-freiem DMEM versetzt wird. Erfindungsgemäß ist somit ferner bevorzugt, dass das Nukleinsäuremolekül mit Polyethylenimin komplexiert ist. In dieser Ausführungsform wird das Polyethylenimin vor der Zugabe zu den Zellen mit dem Nukleinsäuremolekül in Kontakt gebracht und so ein Komplex zwischen den beiden Komponenten gebildet. Der Komplex aus Nukleinsäure und Polyethylenimin wird anschließend zur Zellkultur gegeben. Diese Vorgehensweise erlaubt relativ niedrige Nukleinsäurekonzentrationen im Bereich von etwa 0,1 µg/ml für eine erfolgreiche Transfektion einzusetzen, was natürlich nicht ausschließt, dass auch höhere Konzentrationen von beispielsweise mindestens 0,2 µg/ml, oder mindestens 0,5 µg/ml wie mindestens 1 µg/ml einsetzbar sind. Alternativ dazu und weniger bevorzugt können die beiden Komponenten separat zur Zellkultur gegeben werden.

Bevorzugt ist auch ein Verfahren, wobei das Nukleinsäuremolekül Bestandteil eines Expressionsvektors ist.
In den im erfindungsgemäßen Verfahren verwendeten Vektoren liegen die erfindungsgemäßen Nukleinsäuren in operativer Verknüpfung mit einer Expressionskontrollsequenz vor, so dass sie in einer geeigneten Wirtszelle transkribiert und vorzugsweise translatiert werden können. Expressionskontrollsequenzen umfassen üblicherweise einen Promotor und gegebenenfalls regulatorische Sequenzen wie Operatoren oder Enhancer. Weiterhin können auch Translations-Initiationssequenzen vorhanden sein. Geeignete Expressionskontrollsequenzen für eukaryontische Wirtszellen sind dem Fachmann bekannt (siehe z.B. Sambrook et al., supra). Der erfindungsgemäße rekombinante Vektor kann weiterhin noch übliche Elemente wie einen Replikationsursprung und ein Selektionsmarkergen enthalten. Beispiele für geeignete rekombinante Vektoren, z.B. Plasmide, Cosmide, Phagen, Viren etc., sind dem Fachmann bekannt (siehe z.B. Sambrook et al., supra). Ausgangsmaterialien für die Herstellung der erfindungsgemäßen rekombinanten Vektoren sind kommerziell erhältlich (z.B. von den Firmen Stratagene, InVitroGen oder Promega).

In einer anderen bevorzugten Ausführungsform betriftt die Erfindung ein Verfahren, ferner umfassend den Schritt:
(d) Aufreinigung des produzierten (Poly)peptids.

Die Aufreinigung des so erzeugten (Poly)peptids kann mit üblichen Verfahren erfolgen, die Präzipitationen, z.B. mit Ammoniumsulfat, chromatographische Verfahren etc. einschließen. Chromatographische Verfahren schließen adsorptionschromatographische Verfahren ein, mit denen beispielsweise ein Tag, wie His-tag, Flag-Tag, myc-Tag oder andere Tags, die das erzeugte (Poly)peptid enthalten kann, spezifisch an das chromatographische Material bindet und die (Poly)peptide so aufgereinigt werden. Wenn das so erzeugte (Poly)peptid einen derartigen Tag aufweist, wird dieser nach der Aufreinigung vorzugsweise entfernt. Dies kann z.B. dadurch geschehen, dass der Tag mittels einer ein spezifisches Aminosäuremotiv erkennenden Protease abgespalten wird. Entsprechende Proteasen sind im Stand der Technik bekannt.
Vorzugsweise erfolgt die Herstellung und Aufreinigung der Proteine nach GMP Standards und weiteren nach für die Arzneimittelzulassung vorgeschriebenen Verordnungen und Gesetzen.

Die Erfindung betrifft in einer besonders bevorzugten Ausführungsform ein Verfahren, ferner umfassend den Schritt:
(e) Formulierung des aufgereinigten (Poly)peptids oder eines Fragments oder Derivats davon mit einem pharmazeutisch verträglichen Träger, Exzipienten oder Verdünnungsmittel.

Beispiele für geeignete pharmazeutisch verträgliche Träger, Exzipienten und/oder Verdünnungsmittel sind dem Fachmann bekannt und umfassen z.B. Phosphatgepufferte Kochsalzlösungen, Wasser, Emulsionen, wie z.B. ÖI/Wasser-Emulsionen, verschiedene Arten von Netzmittel oder Detergenzien, sterile Lösungen, etc. Arzneimittel, die solche Träger umfassen, können mittels bekannter konventioneller Methoden formuliert werden. Die so erhaltenen Arzneimittel können einem Individuum in einer geeigneten Dosis verabreicht werden. Die Verabreichung kann oral oder parenteral erfolgen, z.B. intravenös, intraperitoneal, subcutan, intramuskulär, lokal, intranasal, intrabronchial, oral oder intradermal, oder über einen Katheter an einer Stelle in einer Arterie. Präparate für eine parenterale Verabreichung umfassen sterile wäßrige oder nicht-wäßrige Lösungen, Suspensionen und Emulsionen. Beispiele für nicht-wäßrige Lösungsmittel sind Propylenglykol, Polyethylenglykol, pflanzliche Öle wie z.B. Olivenöl, und organische Esterverbindungen wie z.B. Ethyloleat, die für Injektionen geeignet sind. Wäßrige Träger umfassen Wasser, alkoholischwäßrige Lösungen, Emulsionen, Suspensionen, Salzlösungen und gepufferte Medien. Parenterale Träger umfassen Natriumchlorid-Lösungen, Ringer-Dextrose, Dextrose und Natriumchlorid, Ringer-Laktat und gebundene Öle. Intravenöse Träger umfassen z.B. Flüssigkeits-, Nährstoff- und Elektrolyt-Ergänzungsmittel wie z.B. solche, die auf Ringer-Dextrose basieren. Das Arzneimittel kann außerdem Konservierungsmittel und andere Zusätze umfassen, wie z.B. antimikrobielle Verbindungen, Antioxidantien, Komplexbildner und inerte Gase. Des weiteren können, abhängig von der beabsichtigten spezifischen Verwendung, andere Wirkstoffe wie z.B. Interleukine, Wachstumsfaktoren, Differenzierungsfaktoren, Interferone, chemotaktische Proteine oder ein unspezifisches immunmodulatorisches Agens enthalten sein.
Die Art der Dosierung wird vom behandelnden Arzt entsprechend den klinischen Faktoren bestimmt. Es ist dem Fachmann bekannt, daß die Art der Dosierung von verschiedenen Faktoren abhängig ist, wie z.B. der Körpergröße bzw. dem Gewicht, der Körperoberfläche, dem Alter, dem Geschlecht oder der allgemeinen Gesundheit des Patienten, aber auch von dem speziell zu verabreichenden Mittel, der Dauer und Art der Verabreichung, und von anderen Medikamenten, die möglicherweise parallel verabreicht werden. Eine typische Dosis kann z.B. in einem Bereich zwischen 0,001 und 1000 µg liegen, wobei Dosen unterhalb oder oberhalb dieses beispielhaften Bereiches, vor allem unter Berücksichtigung der oben erwähnten Faktoren, vorstellbar sind. Im allgemeinen sollte sich bei regelmäßiger Verabreichung der erfindungsgemäßen Zusammensetzung die Dosis in einem Bereich zwischen 1 µg- und 10 mg-Einheiten pro Tag befinden. Üblicherweise werden die Wirkstoffe in diesen Zubereitungen in einer Konzentration von größer als 10 µg/ml eines physiologischen Puffers vorliegen. Sie können aber auch in fester Form in einer Konzentration von 0,1 bis 99,5 Gew.% der Gesamtmischung vorhanden sein. Im allgemeinen hat es sich als vorteilhaft erwiesen, den oder die Wirkstoffe in Gesamtmengen von etwa 0,001 bis 100 mg/kg, bevorzugt in Gesamtmengen von etwa 0,01 bis 10 mg/kg Körpergewicht je 24 Stunden, gegebenenfalls als Dauerinfusion oder in Form von mehreren Einzelgaben, zur Erzielung des gewünschten Ergebnisses zu verabreichen. Wird die Zusammensetzung intravenös verabreicht, sollte sich die Dosis in einem Bereich zwischen 1 µg- und 10 mg-Einheiten pro Kilogramm Körpergewicht pro Tag befinden. Das Arzneimittel kann topisch, lokal oder systemisch verabreicht werden.

Schließlich betrifft die Erfindung ein Kulturmedium, das das aus Calcium-freiem, mit Vitaminen und Aminosäuren supplementiertem DMEM und Freestyle™ Medium in einem Volumenverhältnis von 30:70 bis 70:30 besteht.

Für das erfindungsgemäße Kulturmedium treffen die für das erfindungsgemäße Verfahren dargestellten (besonders) bevorzugten Ausführungsformen sinngemaß zu und sind sind ebenfalls von der Erfindung umfasst. Das erfindungsgemäße Kulturmedium wird vorteilhafterweise im erfindungsgemäßen Verfahren eingesetzt.

### Die Figuren zeigen:

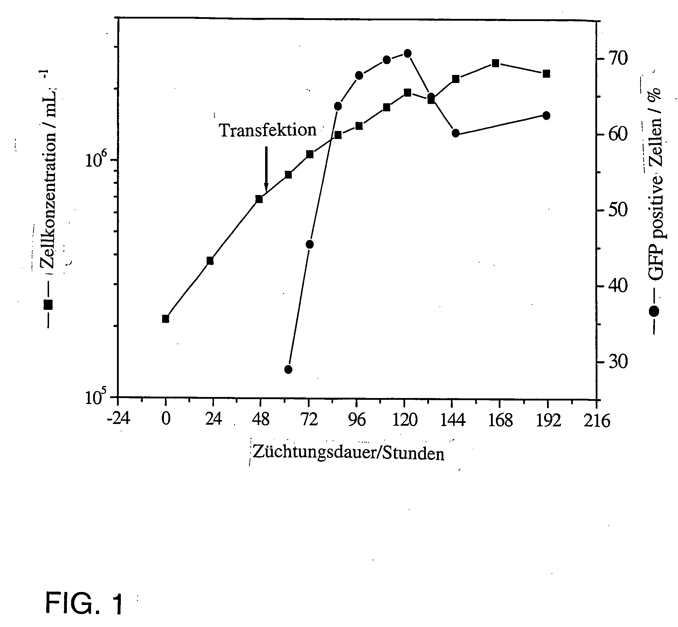

**Fig. 1**: Expression nach transienter Transfektion (vgl. Beispiel 1)

Die Beispiele erläutern die Erfindung.

### Beispiel 1: Transfektion von HEK293s-Zellen mit einem bicistronischen Vektor

Zur Entwicklung des Verfahrens zur transienten Transfektion von HEK293s-Zellen wurde ein Modellproduktionssystem etabliert. Dazu wurden anfangs zwei verschiedene bicistronische Plasmide konstruiert, die das Zielgen (humanes Erythropoetin) in der ersten Position und ein Reportergen (Enhanced Green Fluorescence Protein) in der zweiten Position und umgekehrt enthalten. Unter Verwendung des Polykationen-vermitteltem Gentransfers mit Polyethylenimin (PEI) in Gegenwart einer 1:1 Mischung von Calcium-freiem DMEM und Freestyle Medium konnten wir eine Transfektionseffizienz von bis zu 75 % mit Hilfe des Reportergens in der zweiten Position durch FACScan-Analyse nachweisen (Fig. 1).

## Patentansprüche

1. Verfahren zur transienten Expression von (Poly)peptiden in Säugerzellen, wobei man die folgenden Schritte durchführt:
(a) Züchtung von Säugerzellen in einem Medium, das aus Calcium-freiem, mit Vitaminen und Aminosäuren supplementiertem DMEM und Freestyle™ Medium in einem Volumenverhältnis von 30:70 bis 70:30 besteht;
(b) Transfektion der in Schritt (a) genannten Säugerzellen mit einem das (Poly)peptid kodierenden und exprimierbaren Nucleinsäuremolekül in Calcium-freiem DMEM unter Verwendung von Polyethylenimin; und
(c) Züchtung der in Schritt (b) transfizierten Säugerzellen.

2. Verfahren nach Anspruch 1, wobei die Säugerzellen Teil einer Zelllinie sind.

3. Verfahren nach Anspruch 2, wobei die Zelllinie eine von tierischen Zellen abgeleitete Zelllinie ist.

4. Verfahren nach einem der Ansprüche 1 bis 3, wobei die (Poly)peptide menschliche (Poly)peptide sind.

5. Verfahren nach einem der Ansprüche 1 bis 4, wobei die Transfektion in Schritt (b) in einem Pilot- oder Produktionsbioreaktor durchgeführt wird.

6. Verfahren nach einem der Ansprüche 1 bis 5, wobei das Polyethylenimin ein unverzweigtes Polyethylenimin ist.

7. Verfahren nach einem der Ansprüche 1 bis 6, wobei die Aminosäuren Aspartat und Glutamat sind.

8. Verfahren nach einem der Ansprüche 1 bis 7, wobei das das (Poly)peptid kodierende Nucleinsäuremolekül vor der Transfektion in einem Verhältnis von mindestens 0,5 µg DNA zu 1,5-2 µg/ml Polyethylenimin mit Calcium-freiem DMEM versetzt wird.

9. Verfahren nach einem der Ansprüche 1 bis 8, wobei das Nukleinsäuremolekül mit Polyethylenimin komplexiert ist.

10. Verfahren nach einem der Ansprüche 1 bis 9, wobei das Nukleinsäuremolekül Bestandteil eines Expressionsvektors ist.

11. Verfahren nach einem der Ansprüche 1 bis 10, ferner umfassend den Schritt:
(f) Aufreinigung des produzierten (Poly)peptids.

12. Verfahren nach Anspruch 11, ferner umfassend den Schritt:
(g) Formulierung des aufgereinigten (Poly)peptids oder eines Fragments oder Derivats davon mit einem pharmazeutisch verträglichen Träger, Exzipienten oder Verdünnungsmittel.

13. Kulturmedium, das das aus Calcium-freiem, mit Vitaminen und Aminosäuren supplementiertem DMEM und Freestyle™ Medium in einem Volumenverhältnis von 30:70 bis 70:30 besteht.
